(19) Europäisches Patentamt
European Patent Office
Office européen des brevets

(11) Veröffentlichungsnummer: **0 537 543 A1**

## (12) EUROPÄISCHE PATENTANMELDUNG

(21) Anmeldenummer: **92116617.9**

(22) Anmeldetag: **29.09.92**

(51) Int. Cl.5: **C07D 285/12**, C07D 417/12, A01N 43/82

(30) Priorität: **12.10.91 DE 4133827**

(43) Veröffentlichungstag der Anmeldung:
**21.04.93 Patentblatt 93/16**

(84) Benannte Vertragsstaaten:
**BE CH DE ES FR GB IT LI NL**

(71) Anmelder: **BAYER AG**

**W-5090 Leverkusen 1 Bayerwerk(DE)**

(72) Erfinder: **Förster, Heinz, Dr.**
**Am Eckbusch 47**
**W-5600 Wuppertal 1(DE)**
Erfinder: **Haas, Wilhelm, Dr.**
**Nordstrasse 1**
**W-5014 Kerpen 3(DE)**
Erfinder: **Diehr, Hans-Joachim, Dr.**
**Höhe 35**
**W-5600 Wuppertal 11(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

## (54) 2-(2-(Fluorphenyl)-1,3,4-thiadiazol-5-yl-oxy)-acetamide verwendbar als Herbizide.

(57) Die Erfindung betrifft neue 2-[2-(Fluorphenyl)-1,3,4-thiadiazol-5-yl-oxy]-acetamide der Formel (I)

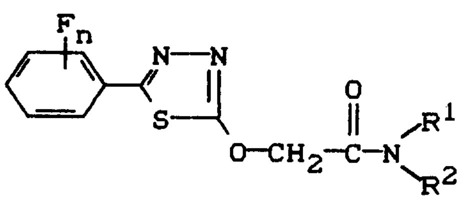

(I)

in welcher

| | |
|---|---|
| $R^1$ | für jeweils gegebenenfalls substituiertes Alkyl, Alkenyl, Alkinyl oder Aralkyl steht und |
| $R^2$ | für jeweils gegebenenfalls substituiertes Alkyl, Alkenyl, Alkinyl, Cycloalkyl, Cycloalkenyl, Alkoxy, Alkenyloxy, Alkinyloxy, Aralkyl oder Aryl steht oder |
| $R^1$ und $R^2$ | gemeinsam mit dem Stickstoffatom, an welches sie gebunden sind, für einen gegebenenfalls substituierten Heterocyclus stehen, der gegebenenfalls weitere Heteroatome enthalten kann, und |
| n | für eine Zahl 1 oder 2 steht, |

ein Verfahren zu ihrer Herstellung und ihre Verwendung als Herbizide.

EP 0 537 543 A1

Die Erfindung betrifft neue 2-[2-(Fluorphenyl)-1,3,4-thiadiazol-5-yl-oxy]-acetamide, ein Verfahren zu ihrer Herstellung und ihre Verwendung als Herbizide.

Es ist bekannt, daß bestimmte 2-Heteroaryloxy-acetamide wie beispielsweise die Verbindung 2-(2-Benzthiazolyloxy)-N-methyl-acetanilid herbizide Eigenschaften besitzen (vergleiche z. B. EP-A 5 501).

Bestimmte 2-(2-Phenyl-1,3,4-thiadiazol-5-yl-oxy)-acetamide, jedoch ohne Fluorsubstituenten an der 2-Phenylgruppe, sind gleichfalls vorbekannt (vgl. z.B. E-A 18 497/US-A 4 645 525 und 4 756 741; EP-A 29 171/US-A 4 408 055; DE-A-30 38 635; EP-A 60 426/US-A-4 465 504).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue 2-[2-(Fluorphenyl)-1,3,4-thiadiazol-5-yl-oxy]-acetamide der allgemeinen Formel (I),

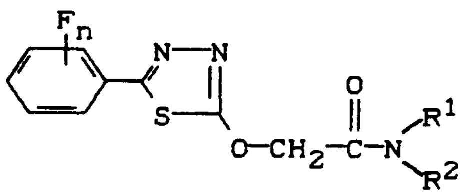

(I)

in welcher

| | |
|---|---|
| $R^1$ | für jeweils gegebenenfalls substituiertes Alkyl, Alkenyl, Alkinyl oder Aralkyl steht und |
| $R^2$ | für jeweils gegebenenfalls substituiertes Alkyl, Alkenyl, Alkinyl, Cycloalkyl, Cycloalkenyl, Alkoxy, Alkenyloxy, Alkinyloxy, Aralkyl oder Aryl steht oder |
| $R^1$ und $R^2$ | gemeinsam mit dem Stickstoffatom, an welches sie gebunden sind, für einen gegebenenfalls substituierten Heterocyclus stehen, der gegebenenfalls weitere Heteroatome enthalten kann, und |
| n | für eine Zahl 1 oder 2 steht, |

gefunden.

Weiterhin wurde gefunden, daß man die neuen 2-[2-(Fluorphenyl)-1,3,4-thiadiazol-5-yl-oxy]-acetamide der allgemeinen Formel (I),

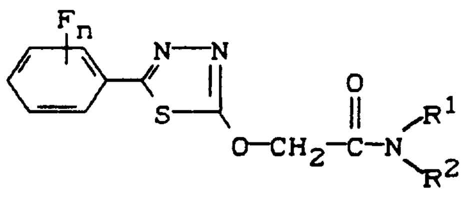

(I)

in welcher

| | |
|---|---|
| $R^1$ | für jeweils gegebenenfalls substituiertes Alkyl, Alkenyl, Alkinyl oder Aralkyl steht und |
| $R^2$ | für jeweils gegebenenfalls substituiertes Alkyl, Alkenyl, Alkinyl, Cycloalkyl, Cycloalkenyl, Alkoxy, Alkenyloxy, Alkinyloxy, Aralkyl oder Aryl steht oder |
| $R^1$ und $R^2$ | gemeinsam mit dem Stickstoffatom, an welches sie gebunden sind, für einen gegebenenfalls substituierten Heterocyclus stehen, der gegebenenfalls weitere Heteroatome enthalten kann, und |
| n | für eine Zahl 1 oder 2 steht, |

erhält, wenn man Fluorphenyl-1,3,4-thiadiazole der Formel (II),

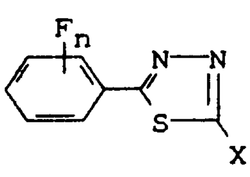

(II)

in welcher

X für Halogen oder Methylsulfonyl steht und

n die oben angegebene Bedeutung hat,

mit 2-Hydroxyacetamiden der Formel (III),

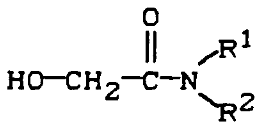

(III)

in welcher

$R^1$ und $R^2$ die oben angegebene Bedeutung haben,

gegebenenfalls in Gegenwart eines Verdünnungsmittels und gegebenenfalls in Gegenwart eines Reaktionshilfsmittels umsetzt.

Schließlich wurde gefunden, daß die neuen 2-[2-(Fluorphenyl)-1,3,4-thiadiazol-5-yl-oxy)-acetamide der allgemeinen Formel (I) herbizide Eigenschaften besitzen.

Überraschenderweise zeigen die erfindungsgemäßen 2-[2-(Fluorphenyl)-1,3,4-thiadiazol-5-yl-oxy]-acetamide der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten, strukturverwandten 2-Heteroaryloxy-acetamiden, wie beispielsweise 2-(2-Benzthiazolyloxy)-N-methyl-acetanilid.

Die erfindungsgemäßen 2-[2-(Fluorphenyl)-1,3,4-thiadiazol-5-yl-oxy]-acetamide sind durch die Formel (I) allgemein definiert. Bevorzugt sind Verbindungen der Formel (I), bei welchen

$R^1$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Cyanalkyl mit 2 bis 8 Kohlenstoffatomen, Alkoxyalkyl mit jeweils 1 bis 8 Kohlenstoffatomen in den einzelnen Alkylteilen, Halogenalkenyl mit 2 bis 8 Kohlenstoffatomen und 1 bis 15 gleichen oder verschiedenen Halogenatomen oder für im Arylteil gegebenenfalls einfach oder mehrfach, gleich oder verschieden substituiertes Aralkyl mit 1 bis 8 Kohlenstoffatomen im geradkettigen oder verzweigten Alkylteil und 6 bis 10 Kohlenstoffatomen im Arylteil steht, wobei als Arylsubstituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl; und

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Cyanalkyl mit 2 bis 8 Kohlenstoffatomen, Alkoxyalkyl mit jeweils 1 bis 8 Kohlenstoffatomen in den einzelnen Alkylteilen, Halogenalkenyl mit 2 bis 8 Kohlenstoffatomen und 1 bis 15 gleichen oder verschiedenen Halogenatomen, Alkoxy mit 1 bis 8 Kohlenstoffatomen, Alkoxyalkoxy mit jeweils 1 bis 8 Kohlenstoffatomen in den einzelnen Alkylteilen, Alkenyloxy mit 2 bis 8 Kohlenstoffatomen oder für jeweils gegebenenfalls einfach oder mehrfach, gleich oder verschieden substituiertes Cycloalkyl oder Cycloalkenyl mit jeweils 3 bis 7 Kohlenstoffatomen steht, wobei als Substituenten jeweils infrage kommen: Halogen sowie geradkettiges oder verzweigtes Alkyl mit 1 bis 4 Kohlenstoffatomen;

und außerdem für jeweils im Arylteil gegebenenfalls einfach oder mehrfach, gleich oder verschieden substituiertes Aralkyl mit 1 bis 8 Kohlenstoffatomen im geradkettigen oder verzweigten Alkylteil und 6 bis 10 Kohlenstoffatomen im Arylteil oder Aryl mit 6 bis 10 Kohlenstoffatomen steht, wobei als Arylsubstituenten jeweils die bei $R^1$ genannten infrage kommen, oder

$R^1$ und $R^2$ gemeinsam mit dem Stickstoffatom, an welches sie gebunden sind, für einen gesättigten

oder ungesättigten, gegebenenfalls benzoannellierten Heterocyclus mit 2 bis 9 Kohlenstoffatomen und 1 bis 4 gleichen oder verschiedenen Heteroatomen - insbesondere Stickstoff, Sauerstoff und/oder Schwefel - stehen, welcher gegebenenfalls einfach oder mehrfach, gleich oder verschieden durch geradkettiges oder verzweigtes Alkyl mit 1 bis 4 Kohlenstoffatomen substituiert ist, und

n für eine Zahl 1 oder 2 steht.

Besonders bevorzugt sind Verbindungen der Formel (I), bei welchen

$R^1$ für jeweils geradkettiges oder verzweigtes Alkyl mit 1 bis 4 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Cyanalkyl mit 2 bis 4 Kohlenstoffatomen, Alkoxyalkyl mit jeweils 1 bis 4 Kohlenstoffatomen in den einzelnen Alkylteilen, Halogenalkenyl mit 3 bis 6 Kohlenstoffatomen und 1 bis 5 gleichen oder verschiedenen Halogenatomen oder für im Arylteil gegebenenfalls einfach bis dreifach, gleich oder verschieden substituiertes Aralkyl mit 1 bis 4 Kohlenstoffatomen im geradkettigen oder verzweigten Alkylteil und 6 oder 10 Kohlenstoffatomen im Arylteil steht, wobei als Arylsubstituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis dreifach, gleich oder verschieden durch Halogen und/oder geradkettiges oder verzweigtes Alkyl mit 1 bis 3 Kohlenstoffatomen substituiertes Phenyl; und

$R^2$ für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Cyanalkyl mit 2 bis 4 Kohlenstoffatomen, Alkoxyalkyl mit jeweils 1 bis 4 Kohlenstoffatomen in den einzelnen Alkylteilen, Halogenalkenyl mit 3 bis 6 Kohlenstoffatomen und 1 bis 5 gleichen oder verschiedenen Halogenatomen, Alkoxy mit 1 bis 6 Kohlenstoffatomen, Alkoxyalkoxy mit jeweils 1 bis 4 Kohlenstoffatomen in den einzelnen Alkylteilen, Alkenyloxy mit 3 bis 4 Kohlenstoffatomen oder für jeweils gegebenenfalls einfach bis vierfach, gleich oder verschieden substituiertes Cycloalkyl oder Cycloalkenyl mit jeweils 5 bis 7 Kohlenstoffatomen steht, wobei als Substituenten jeweils infrage kommen: Halogen sowie geradkettiges oder verzweigtes Alkyl mit 1 bis 3 Kohlenstoffatomen;

und außerdem für jeweils im Arylteil gegebenenfalls einfach bis dreifach, gleich oder verschieden substituiertes Aralkyl mit 1 bis 4 Kohlenstoffatomen im geradkettigen oder verzweigten Alkylteil und 6 oder 10 Kohlenstoffatomen im Arylteil oder Aryl mit 6 oder 10 Kohlenstoffatomen steht, wobei als Arylsubstituenten jeweils die bei $R^1$ genannten infrage kommen, oder

$R^1$ und $R^2$ gemeinsam mit dem Stickstoffatom, an welches sie gebunden sind, für einen gesättigten oder ungesättigten, gegebenenfalls benzoannellierten Heterocyclus mit 2 bis 5 Kohlenstoffatomen und 1 bis 3 gleichen oder verschiedenen Heteroatomen - insbesondere Stickstoff, Sauerstoff und/oder Schwefel - stehen, welcher gegebenenfalls einfach bis dreifach, gleich oder verschieden durch geradkettiges oder verzweigtes Alkyl mit 1 bis 3 Kohlenstoffatomen substituiert ist, und

n für eine Zahl 1 oder 2 steht.

Ganz besonders bevorzugt sind Verbindungen der Formel (I), bei welchen

$R^1$ für Methyl, Ethyl, n- oder i-Propyl, n-, i-, s- oder t-Butyl, Allyl, Propargyl oder für gegebenenfalls einfach oder zweifach substituiertes Benzyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis zweifach, gleich oder verschieden durch Fluor, Chlor, Brom, Methyl und/oder Ethyl substituiertes Phenyl, und

$R^2$ für jeweils geradkettiges oder verzweigtes Alkyl oder Alkoxy mit jeweils 1 bis 6 Kohlenstoffatomen, Methoxymethyl, Methoxyethyl, Ethoxymethyl, Ethoxyethyl, Methoxymethoxy,

Methoxyethoxy, Ethoxymethoxy, Ethoxyethoxy, für Allyl, Allyloxy, n- oder i-Butenyl, Propargyl, Propargyloxy, n- oder i-Butinyl, für Cyclopentyl, Cyclohexyl, Cyclohexenyl oder für jeweils gegebenenfalls einfach oder zweifach, gleich oder verschieden substituiertes Benzyl oder Phenyl steht, wobei als Substituenten jeweils die bei $R^1$ genannten infrage kommen, oder

$R^1$ und $R^2$ gemeinsam mit dem Stickstoffatom, an welches sie gebunden sind, für jeweils gegebenenfalls einfach bis dreifach, gleich oder verschieden substituiertes Pyrrolidinyl, Piperidinyl, Morpholinyl, Perhydroazepinyl oder 1,2,3,4-Tetrahydrochinolyl stehen, wobei als Substituenten jeweils infrage kommen:
Methyl, Ethyl, n- oder i-Propyl und

n für eine Zahl 1 oder 2 steht.

Im einzelnen sei auf die bei den Herstellungsbeispielen genannten Verbindungen verwiesen.

Verwendet man beispielsweise 2-Chlor-5-(2-fluorphenyl)-1,3,4-thiadiazol und 2-Hydroxy-N-methyl-N-n-butyl-acetamid als Ausgangsstoffe, so läßt sich der Reaktionsablauf des erfindungsgemäßen Verfahrens durch das folgende Formelschema darstellen:

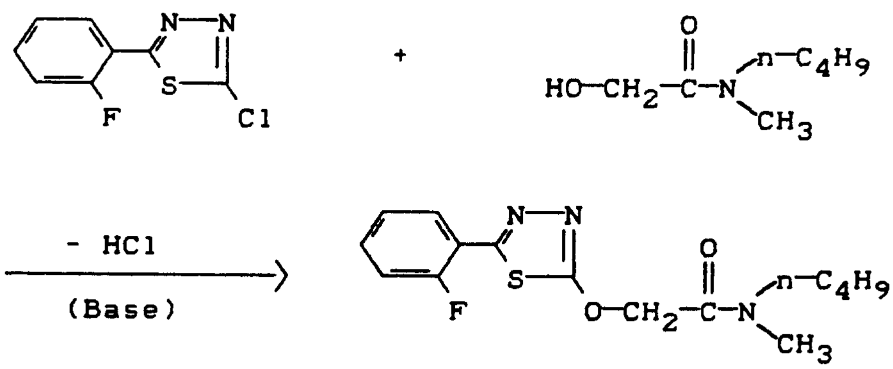


Die zur Durchführung des erfindungsgemäßen Verfahrens als Ausgangsstoffe benötigten Fluorphenyl-1,3,4-thiadiazole sind durch die Formel (II) allgemein definiert. In dieser Formel (II) steht der Index n auch vorzugsweise für eine Zahl 1 oder 2. X steht vorzugsweise für Fluor, Chlor, Brom oder Methylsulfonyl, insbesondere für Chlor. Die Fluorphenyl-1,3,4-thiadiazole der Formel (II) sind bekannt oder erhältlich in Analogie zu bekannten Verfahren (vergleiche z. B. J. Med. Chem. 31, 906-913 [1988]; US 4.454.147; J. Pharm. Sci. 64, 1250-1252 [1975]); EP-A 440 959).

Die zur Durchführung des erfindungsgemäßen Verfahrens weiterhin als Ausgangsstoffe benötigten 2-Hydroxyacetamide sind durch die Formel (III) allgemein definiert.

In dieser Formel (III) stehen $R^1$ und $R^2$ vorzugsweise für diejenigen Reste, die bereits im Zusammenhang mit der Beschreibung der erfindungsgemäßen Verbindungen der Formel (I) als bevorzugt für diese Substituenten genannt wurden.

Die 2-Hydroxyacetamide der Formel (III) sind ebenfalls bekannt (vergleiche z. B. DE 23 10 757; EP 5 501; EP 37 526; US 4.509.971; US 4.645.525; US 4.334.073).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester, Sulfoxide, wie Dimethylsulfoxid oder Alkohole, wie Methanol, Ethanol, n- oder i-Propanol oder n-, i-, s- oder t-Butanol.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie beispielsweise Trimethylamin,

Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen -20 °C und 120 °C, vorzugsweise bei Temperaturen zwischen 0 °C und 60 °C.

Zur Durchführung des erfindungsgemäßen Verfahrens setzt man pro Mol an Fluorphenyl-1,3,4-thiadiazol der Formel (II) im allgemeinen 1,0 bis 2,0 Mol, vorzugsweise 1,0 bis 1,5 Mol 2-Hydroxyacetamid der Formel (III) und gegebenenfalls 0,1 bis 2,0 Mol, vorzugsweise 1,0 bis 1,2 Mol Reaktionshilfsmittel ein.
Die Reaktionsdurchführung, Aufarbeitung und Isolierung der Reaktionsprodukte erfolgt nach bekannten Verfahren (vergleiche hierzu beispielsweise EP 348 734; EP 5 501 sowie die Herstellungsbeispiele).

Die Reinigung der Endprodukte der Formel (I) erfolgt mit Hilfe üblicher Verfahren, beispielsweise durch Säulenchromatographie oder durch Umkristallisieren.
Die Charakterisierung erfolgt mit Hilfe des Schmelzpunktes oder bei nicht kristallisierenden Verbindungen mit Hilfe des Brechungsindex oder der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von monokotylen Unkräutern in monokotylen und dikotylen Kulturen wie beispielsweise Baumwolle oder Reis einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln unter Durck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

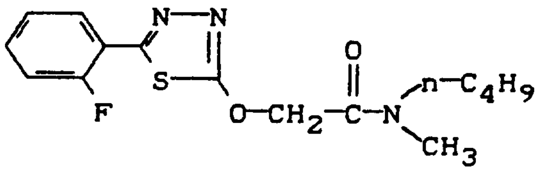

Zu 1,69 g (0,011 Mol) Hydroxyessigsäure-N-methyl-N-n-butylamid und 1,23 g (0,011 Mol) Kalium-tert.-Butylat in 50 ml t-Butanol gibt man bei 20 ° C unter Rühren 2,5 g (0,011 Mol) 2-Chlor-5-(2-fluorphenyl)-1,3,4-thiadiazol und rührt anschließend 12 Stunden bei Raumtemperatur. Zur Aufarbeitung gibt man Wasser zu, rührt so lange, bis das Produkt kristallin wird, saugt ab und trocknet.

Man erhält 2,2 g (54 % der Theorie) 2-[2-(2-Fluorphenyl)-1,3,4-thiadiazol-5-yloxy]-essigsäure-N-methyl-N-n-butylamid vom Schmelzpunkt 92 ° C.

In entspechender Weise und gemäß den allgemeinen Angaben zur Herstellung erhält man die folgenden 2-[2-(Fluorphenyl)-1,3,4-thiadiazol-5-yl-oxy]-acetamide der allgemeinen Formel (I):

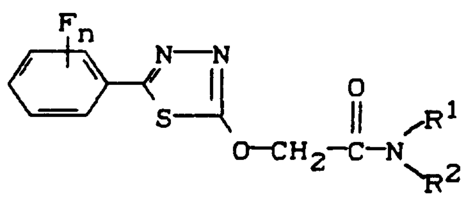

(I)

| Bsp.-Nr. | $R^1$ | $R^2$ | 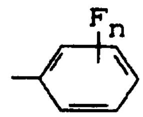 | physikalische Eigenschaften |
|---|---|---|---|---|
| 2 | $i-C_3H_7$ | 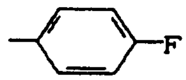 | 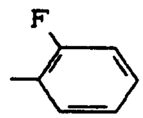 | Fp 137° C |
| 3 | $CH_3$ | $n-C_4H_9$ | 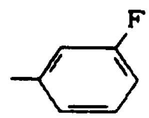 | Fp 74° C |
| 4 | $i-C_3H_7$ | 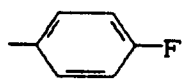 | 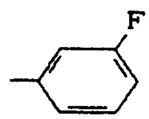 | Fp 125° C |
| 5 | 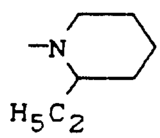 | | 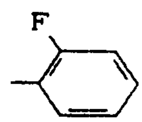 | Fp 98° C |
| 6 | 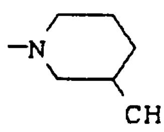 | | 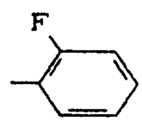 | Fp 99° C |
| 7 | $CH_3$ | $C_6H_5$ | 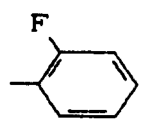 | Fp 111° C |
| 8 | $CH_3$ | 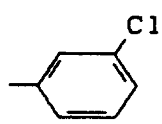 | 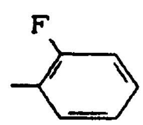 | Fp 148° C |
| 9 | $CH_3$ | 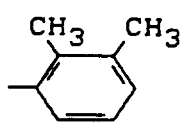 | 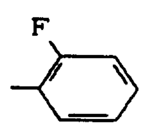 | Fp 140° C |

| Bsp.-Nr. | $R^1$ | $R^2$ | 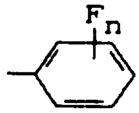 | physikalische Eigenschaften |
|---|---|---|---|---|
| 10 | $-CH_2-CH{=}CH_2$ | $-CH_2-CH{=}CH_2$ | 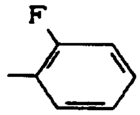 | Fp 85° C |
| 11 | $C_2H_5$ | $C_6H_5$ | 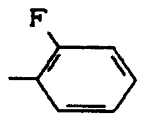 | Fp 125° C |
| 12 | 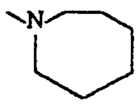 | | 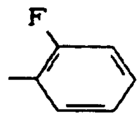 | Fp 111° C |
| 13 | 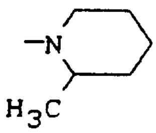 | | 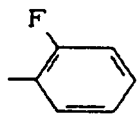 | Fp 90° C |
| 14 | $n-C_4H_9$ | $n-C_4H_9$ | 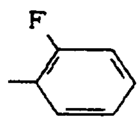 | $n_D^{20}$ 1,5118 |
| 15 | $i-C_3H_7$ | $C_6H_5$ | 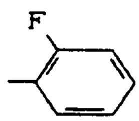 | Fp 128° C |
| 16 | $i-C_3H_7$ | $-O-i-C_3H_7$ | 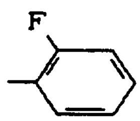 | Fp 110° C |
| 17 | $n-C_3H_7$ | $n-C_3H_7$ | 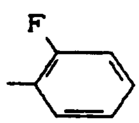 | Fp 106° C |

| Bsp.-Nr. | $R^1$ | $R^2$ | 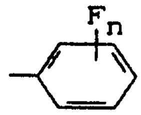 | physikalische Eigenschaften |
|---|---|---|---|---|
| 18 | $i\text{-}C_3H_7$ | 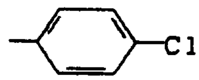 | 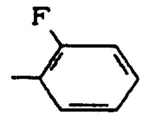 | Fp 116° C |
| 19 | $i\text{-}C_3H_7$ | 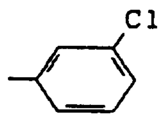 | 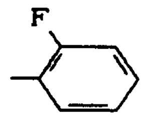 | Fp 128° C |
| 20 | $i\text{-}C_3H_7$ | 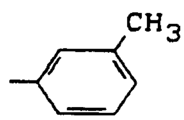 | 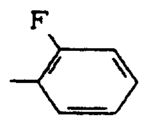 | Fp 108° C |
| 21 | 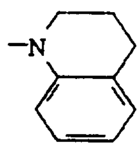 | | 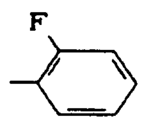 | Fp 93° C |
| 22 | $i\text{-}C_3H_7$ | 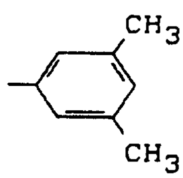 | 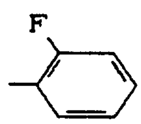 | Fp 94° C |
| 23 | $i\text{-}C_3H_7$ | 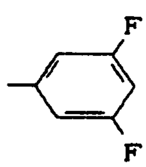 | 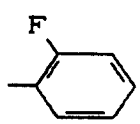 | Fp 99° C |
| 24 | $i\text{-}C_3H_7$ | 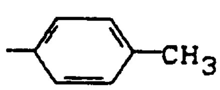 | 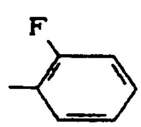 | Fp 106° C |

| Bsp.-Nr. | $R^1$ | $R^2$ | 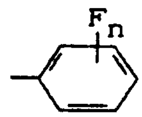 | physikalische Eigenschaften |
|---|---|---|---|---|
| 25 | $CH_3$ | $C_6H_5$ | 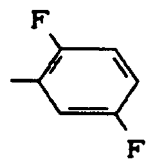 | Fp 138° C |
| 26 | $n-C_3H_7$ | $C_6H_5$ | 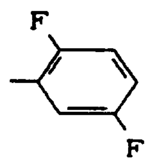 | Fp 140° C |
| 27 | 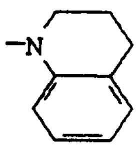 | | 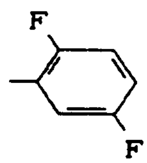 | Fp 132° C |
| 28 | $CH_3$ | 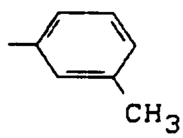 | 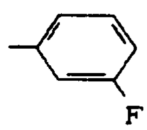 | Fp 132° C |
| 29 | $i-C_3H_7$ | 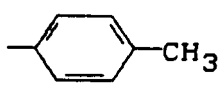 | 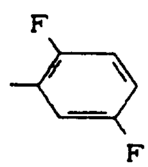 | Fp 161° C |
| 30 | $i-C_3H_7$ | 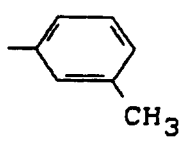 | 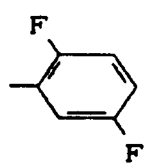 | Fp 118° C |
| 31 | $CH_3$ | 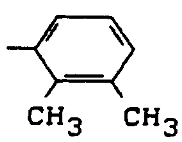 | 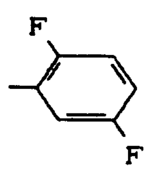 | Fp 158° C |

| Bsp.-Nr. | $R^1$ | $R^2$ | 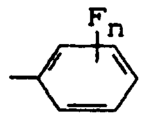 | physikalische Eigenschaften |
|---|---|---|---|---|
| 32 | $-CH_2-CH=CH_2$ | $-CH_2-CH=CH_2$ | 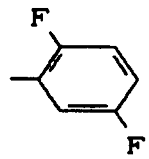 | |
| 33 | $C_2H_5$ | $C_6H_5$ | 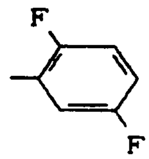 | |
| 34 | 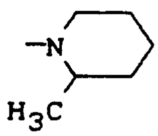 | | 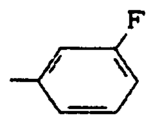 | Fp 78° C |
| 35 | $CH_3$ | $C_6H_5$ | 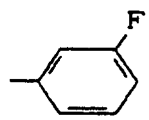 | Fp 121° C |
| 36 | $CH_3$ | 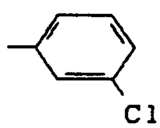 | 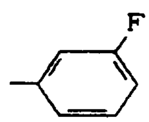 | |
| 37 | 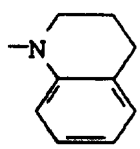 | | 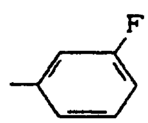 | Fp 98° C |
| 38 | $i-C_3H_7$ | 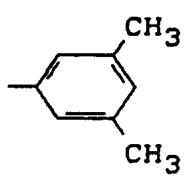 | 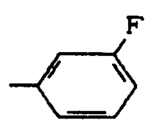 | Fp 88° C |

| Bsp.-Nr. | $R^1$ | $R^2$ | 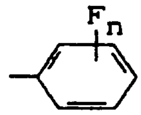 | physikalische Eigenschaften |
|---|---|---|---|---|
| 39 | $i-C_3H_7$ | 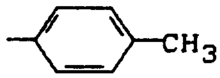 | 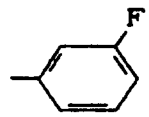 | Fp 108° C |
| 40 | $i-C_3H_7$ | 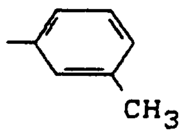 | 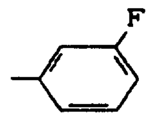 | Fp 104° C |
| 41 | $CH_3$ | 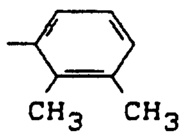 | 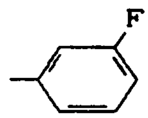 | Fp 120° C |
| 42 | $-CH_2-CH=CH_2$ | $-CH_2-CH=CH_2$ | 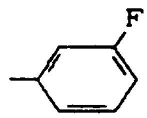 | $n_D^{20}$ 1,5680 |
| 43 | $C_2H_5$ | $C_6H_5$ | 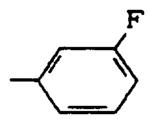 | Fp 116° C |
| 44 | 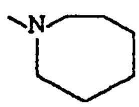 | | 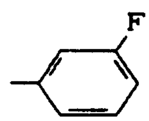 | Fp 128° C |
| 45 | 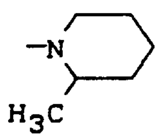 | | 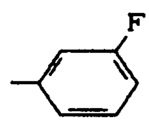 | $n_D^{20}$ 1,5230 |
| 46 | $i-C_3H_7$ | $C_6H_5$ | 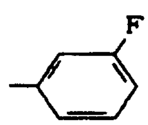 | Fp 140° C |

| Bsp.-Nr. | $R^1$ | $R^2$ | 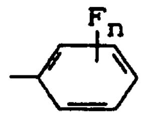 | physikalische Eigenschaften |
|---|---|---|---|---|
| 47 | $C_2H_5$ | $C_2H_5$ | 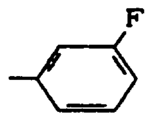 | |
| 48 | $i-C_3H_7$ | $-O-CH_2-CH_2-O-C_2H_5$ | 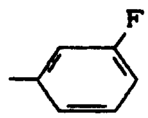 | Fp 58° C |
| 49 | $CH_3$ | 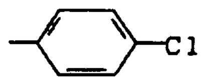 | 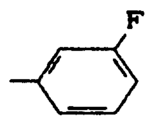 | |
| 50 | $i-C_3H_7$ | 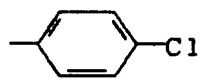 | 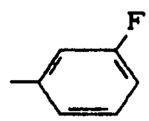 | Fp 102° C |
| 51 | $n-C_3H_7$ | $n-C_3H_7$ | 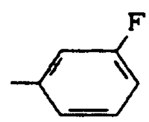 | Fp 88° C |
| 52 | $CH_3$ | $-OCH_3$ | 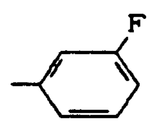 | Fp 80° C |
| 53 | $CH_3$ | 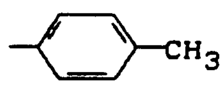 | 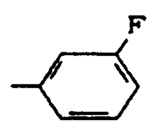 | Fp 94° C |
| 54 | 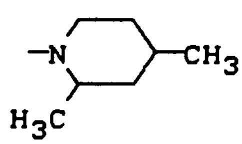 | | 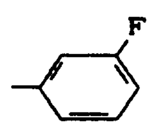 | Fp 68-70° C |

| Bsp.-Nr. | $R^1$ | $R^2$ | 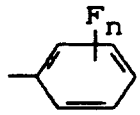 | physikalische Eigenschaften |
|---|---|---|---|---|
| 55 | 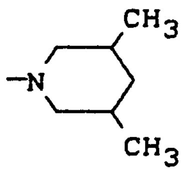 | | 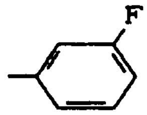 | $n_D^{20}$ 1,5542 |
| 56 | $CH_3$ | 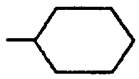 | 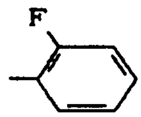 | Fp 110° C |
| 57 | $CH_3$ | 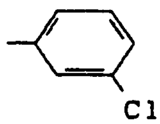 | 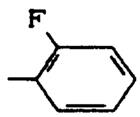 | Fp 118° C |
| 58 | $CH_3$ | $-OCH_3$ | 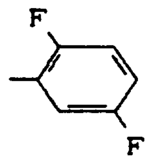 | Fp 104° C |
| 59 | $n-C_4H_9$ | $n-C_4H_9$ | 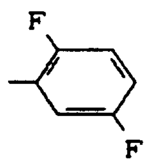 | Fp 75° C |
| 60 | $i-C_3H_7$ | 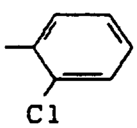 | 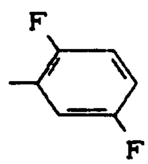 | Fp 103° C |
| 61 | 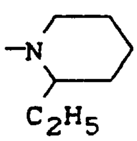 | | 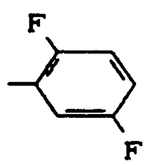 | Fp 97° C |

| Bsp.-Nr. | $R^1$ | $R^2$ | 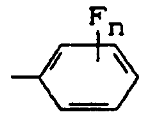 | physikalische Eigenschaften |
|---|---|---|---|---|
| 62 | $CH_3$ | $-OCH_3$ | 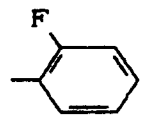 | Fp 83° C |
| 63 | 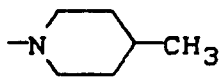 | | 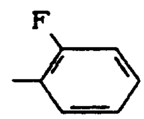 | Fp 113° C |
| 64 | 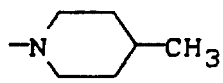 | | 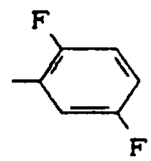 | Fp 160° C |
| 65 | i-$C_3H_7$ | 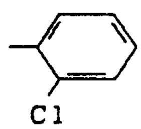 | 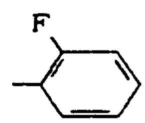 | Fp 104° C |
| 66 | 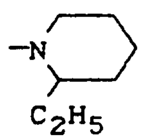 | | 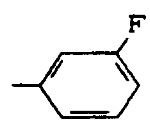 | Wachs |
| 67 | 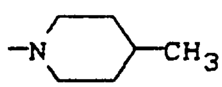 | | 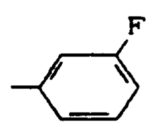 | Fp 139° C |
| 68 | i-$C_3H_7$ | 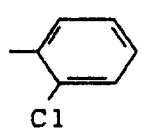 | 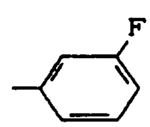 | Fp 83° C |

| Bsp.-Nr. | $R^1$ | $R^2$ | 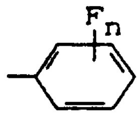 | physikalische Eigenschaften |
|---|---|---|---|---|
| 69 | $n\text{-}C_4H_9$ | $n\text{-}C_4H_9$ | 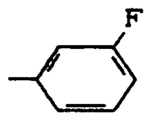 | Fp 41° C |

Anwendungsbeispiele

In den folgenden Anwendungsbeispielen wurde die nachstehend aufgeführte Verbindung als Vergleichssubstanz eingesetzt:

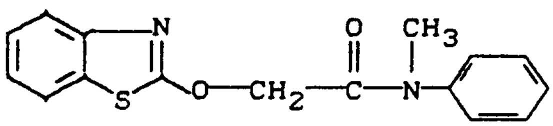

(A)

2-(2-Benzthiazolyloxy)-N-methyl-acetanilid (bekannt aus EP 5 501):

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen, Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigen in diesem Test z.B. die Verbindungen gemäß folgender Herstellungsbeispiele 1, 2, 3 und 4.

Beispiel B

Pre-emergence-Test / Wasseroberflächenbehandlung bei verpflanztem Wasserreis

Zur Herstellung einer applizierfähigen Zubereitung wird ein Teil Wirkstoff mit 5 Teilen Aceton gelöst; anschließend wird ein Teil Benzyloxy-polyglykol-ether als Emulgator zugegeben und das Konzentrat mit Wasser auf die gewünschte Konzentration verdünnt.

In Töpfe, die mit Erde gefüllt sind, wird Reis im 2- bis 3-Blattstadium verpflanzt. Samen von Testpflanzen werden ausgelegt (1 cm tief). Zwei Tage später werden die Töpfe mit Wasser 3 cm überstaut. Anschließend werden die Wirkstoffzubereitungen auf die Wasseroberfläche ausgebracht. 4 Wochen später wird die herbizide Wirkung und die Schädigung der behandelten Pflanzen im Vergleich zu unbehandelten visuell in % ausgewertet.

Es bedeuten:
0 % = keine Wirkung
100 % = völliges Absterben

Eine deutliche Überlegenheit in der Wirksamkeit bei vergleichbarer Nutzpflanzenselektivität gegenüber dem Stand der Technik zeigen in diesem Test z. B. die Verbindungen gemäß den Herstellungsbeispielen 5, 7, 8, 9, 10, 11, 12, 13, 14, 21, 59, 60, 61, 62, 63, 64, 65, 66, 67 und 69.

**Patentansprüche**

**1.** 2-[2-(Fluorphenyl)-1,3,4-thiadiazol-5-yl-oxy]-acetamide der allgemeinen Formel (I)

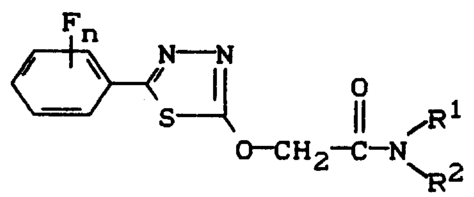

(I)

in welcher

| | |
|---|---|
| $R^1$ | für jeweils gegebenenfalls substituiertes Alkyl, Alkenyl, Alkinyl oder Aralkyl steht und |
| $R^2$ | für jeweils gegebenenfalls substituiertes Alkyl, Alkenyl, Alkinyl, Cycloalkyl, Cycloalkenyl, Alkoxy, Alkenyloxy, Alkinyloxy, Aralkyl oder Aryl steht oder |
| $R^1$ und $R^2$ | gemeinsam mit dem Stickstoffatom, an welches sie gebunden sind, für einen gegebenenfalls substituierten Heterocyclus stehen, der gegebenenfalls weitere Heteroatome enthalten kann, und |
| n | für eine Zahl 1 oder 2 steht. |

**2.** Verbindungen der allgemeinen Formel (I) gemäß Anspruch 1, dadurch gekennzeichnet, daß darin

| | |
|---|---|
| $R^1$ | für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Cyanalkyl mit 2 bis 8 Kohlenstoffatomen, Alkoxyalkyl mit jeweils 1 bis 8 Kohlenstoffatomen in den einzelnen Alkylteilen, Halogenalkenyl mit 2 bis 8 Kohlenstoffatomen und 1 bis 15 gleichen oder verschiedenen Halogenatomen oder für im Arylteil gegebenenfalls einfach oder mehrfach, gleich oder verschieden substituiertes Aralkyl mit 1 bis 8 Kohlenstoffatomen im geradkettigen oder verzweigten Alkylteil und 6 bis 10 Kohlenstoffatomen im Arylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl; und |
| $R^2$ | für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Cyanalkyl mit 2 bis 8 Kohlenstoffatomen, Alkoxyalkyl mit jeweils 1 bis 8 Kohlenstoffatomen in den einzelnen Alkylteilen, Halogenalkenyl mit 2 bis 8 Kohlenstoffatomen und 1 bis 15 gleichen oder verschiedenen Halogenatomen, Alkoxy mit 1 bis 8 Kohlenstoffatomen, Alkoxyalkoxy mit jeweils 1 bis 8 Kohlenstoffatomen in den einzelnen Alkylteilen, Alkenyloxy mit 2 bis 8 Kohlenstoffatomen oder für jeweils gegebenenfalls einfach oder mehrfach, gleich oder verschieden substituiertes Cycloalkyl oder Cycloalkenyl mit jeweils 3 bis 7 Kohlenstoffatomen steht, wobei als Substituenten jeweils infrage kommen: Halogen sowie geradkettiges oder verzweigtes Alkyl mit 1 bis |

4 Kohlenstoffatomen;
und außerdem für jeweils im Arylteil gegebenenfalls einfach oder mehrfach, gleich oder verschieden substituiertes Aralkyl mit 1 bis 8 Kohlenstoffatomen im geradkettigen oder verzweigten Alkylteil und 6 bis 10 Kohlenstoffatomen im Arylteil oder Aryl mit 6 bis 10 Kohlenstoffatomen steht, wobei als Arylsubstituenten jeweils die bei $R^1$ genannten in Frage kommen, oder

$R^1$ und $R^2$ gemeinsam mit dem Stickstoffatom, an welches sie gebunden sind, für einen gesättigten oder ungesättigten, gegebenenfalls benzoannellierten Heterocyclus mit 2 bis 9 Kohlenstoffatomen und 1 bis 4 gleichen oder verschiedenen Heteroatomen - insbesondere Stickstoff, Sauerstoff und/oder Schwefel - stehen, welcher gegebenenfalls einfach oder mehrfach, gleich oder verschieden durch geradkettiges oder verzweigtes Alkyl mit 1 bis 4 Kohlenstoffatomen substituiert ist, und

n für eine Zahl 1 oder 2 steht.

**3.** Verbindungen der allgemeinen Formel (I) gemäß Anspruch 1, dadurch gekennzeichnet, daß darin

$R^1$ für jeweils geradkettiges oder verzweigtes Alkyl mit 1 bis 4 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Cyanalkyl mit 2 bis 4 Kohlenstoffatomen, Alkoxyalkyl mit jeweils 1 bis 4 Kohlenstoffatomen in den einzelnen Alkylteilen, Halogenalkenyl mit 3 bis 6 Kohlenstoffatomen und 1 bis 5 gleichen oder verschiedenen Halogenatomen oder für im Arylteil gegebenenfalls einfach bis dreifach, gleich oder verschieden substituiertes Aralkyl mit 1 bis 4 Kohlenstoffatomen im geradkettigen oder verzweigten Alkylteil und 6 oder 10 Kohlenstoffatomen im Arylteil steht, wobei als Arylsubstituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen sowie gegebenenfalls einfach bis dreifach, gleich oder verschieden durch Halogen und/oder geradkettiges oder verzweigtes Alkyl mit 1 bis 3 Kohlenstoffatomen substituiertes Phenyl; und

$R^2$ für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Cyanalkyl mit 2 bis 4 Kohlenstoffatomen, Alkoxyalkyl mit jeweils 1 bis 4 Kohlenstoffatomen in den einzelnen Alkylteilen, Halogenalkenyl mit 3 bis 6 Kohlenstoffatomen und 1 bis 5 gleichen oder verschiedenen Halogenatomen, Alkoxy mit 1 bis 6 Kohlenstoffatomen, Alkoxyalkoxy mit jeweils 1 bis 4 Kohlenstoffatomen in den einzelnen Alkylteilen, Alkenyloxy mit 3 bis 4 Kohlenstoffatomen oder für jeweils gegebenenfalls einfach bis vierfach, gleich oder verschieden substituiertes Cycloalkyl oder Cycloalkenyl mit jeweils 5 bis 7 Kohlenstoffatomen steht, wobei als Substituenten jeweils infrage kommen: Halogen sowie geradkettiges oder verzweigtes Alkyl mit 1 bis 3 Kohlenstoffatomen;
und außerdem für jeweils im Arylteil gegebenenfalls einfach bis dreifach, gleich oder verschieden substituiertes Aralkyl mit 1 bis 4 Kohlenstoffatomen im geradkettigen oder verzweigten Alkylteil und 6 oder 10 Kohlenstoffatomen im Arylteil oder Aryl mit 6 oder 10 Kohlenstoffatomen steht, wobei als Arylsubstituenten jeweils die bei $R^1$ genannten infrage kommen, oder

$R^1$ und $R^2$ gemeinsam mit dem Stickstoffatom, an welches sie gebunden sind, für einen gesättigten oder ungesättigten, gegebenenfalls benzoannellierten Heterocyclus mit 2 bis 5 Kohlenstoffatomen und 1 bis 3 gleichen oder verschiedenen Heteroatomen - insbesondere Stickstoff, Sauerstoff und/oder Schwefel - stehen, welcher gegebenenfalls einfach bis dreifach, gleich oder verschieden durch geradkettiges oder verzweigtes Alkyl mit 1 bis 3 Kohlenstoffatomen substituiert ist, und

n für eine Zahl 1 oder 2 steht.

4. Verbindungen der allgemeinen Formel (I) gemäß Anspruch 1, dadurch gekennzeichnet, daß darin

$R^1$ für Methyl, Ethyl, n- oder i-Propyl, n-, i-, s-oder t-Butyl, Allyl, Propargyl oder für gegebenenfalls einfach oder zweifach substituiertes Benzyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethox-Iminomethyl, Ethoximinoethyl oder gegebenenfalls ein- bis zweifach, gleich oder verschieden durch Fluor, Chlor, Brom, Methyl und/oder Ethyl substituiertes Phenyl, und

$R^2$ für jeweils geradkettiges oder verzweigtes Alkyl oder Alkoxy mit jeweils 1 bis 6 Kohlenstoffatomen, Methoxymethyl, Methoxyethyl, Ethoxymethyl, Ethoxyethyl, Methoxymethoxy, Methoxyethoxy, Ethoxymethoxy, Ethoxyethoxy, für Allyl, Allyloxy, n- oder i-Butenyl, Propargyl, Propargyloxy, n-oder i-Butinyl, für Cyclopentyl, Cyclohexyl, Cyclohexenyl oder für jeweils gegebenenfalls einfach oder zweifach, gleich oder verschieden substituiertes Benzyl oder Phenyl steht, wobei als Substituenten jeweils die bei $R^1$ genannten infrage kommen, oder

$R^1$ und $R^2$ gemeinsam mit dem Stickstoffatom, an welches sie gebunden sind, für jeweils gegebenenfalls einfach bis dreifach, gleich oder verschieden substituiertes Pyrrolidinyl, Piperidinyl, Morpholinyl, Perhydroazepinyl oder 1,2,3,4-Tetrahydrochinolyl stehen, wobei als Substituenten jeweils infrage kommen: Methyl, Ethyl, n- oder i-Propyl und

n für eine Zahl 1 oder 2 steht.

5. Verfahren zur Herstellung von 2-[2-(Fluorphenyl)-1,3,4-thiadiazol-5-yl-oxy]-acetamiden der allgemeinen Formel (I),

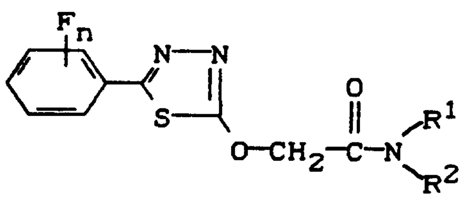

(I)

in welcher

$R^1$ für jeweils gegebenenfalls substituiertes Alkyl, Alkenyl, Alkinyl oder Aralkyl steht und

$R^2$ für jeweils gegebenenfalls substituiertes Alkyl, Alkenyl, Alkinyl, Cycloalkyl, Cycloalkenyl, Alkoxy, Alkenyloxy, Alkinyloxy, Aralkyl oder Aryl steht oder

$R^1$ und $R^2$ gemeinsam mit dem Stickstoffatom; Stickstoffatom, an welches die gebunden sind, für einen gegebenenfalls substituierten Heterocyclus stehen, der gegebenenfalls weitere Heteroatome enthalten kann, und

n für eine Zahl 1 oder 2 steht,

dadurch gekennzeichnet, daß man Fluorphenyl-1,3,4-thiadiazole der Formel (II),

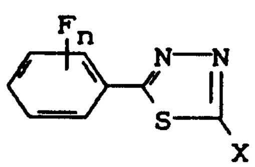

(II)

in welcher

X für Halogen doer Methylsulfonyl steht und

n die oben angegebene Bedeutung hat,

mit 2-Hydroxyacetamiden der Formel (III)

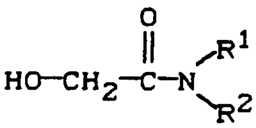

(III)

in welcher

$R^1$ und $R^2$ die oben angegebene Bedeutung haben,

gegegebenenfalls in Gegenwart eines Verdünnungsmittels und gegebenenfalls in Gegenwart eines Reaktionshilfsmittels umsetzt.

6. Herbizide Mittel, gekennzeichnet durch einen Gehalt an mindestens einer Verbindung der Formel (I) gemäß Anspruch 1.

7. Verwendung von Verbindungen der allgemeinen Formel (I) gemäß Anspruch 1 zur Bekämpfung von unerwünschtem Pflanzenwachstum.

8. Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

9. Verfahren zur Herstellung von herbiziden Mitteln, dadurch gekennzeichnet, daß man Verbindungen der allgemeinen Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

EP 92 11 6617

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | EP-A-0 018 497 (BAYER AG)<br>* das ganze Dokument, insbesondere Seite 6, Zeilen 32-34, Seite 10, Seiten 64-67, Verbindungen 230-233 und 245 *<br>--- | 1-9 | C07D285/12<br>C07D417/12<br>A01N43/82 |
| D,A | DE-A-3 038 635 (BAYER AG)<br>* das ganze Dokument *<br>----- | 1-9 | |
| | | | **RECHERCHIERTE SACHGEBIETE (Int. Cl.5)** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 08 JANUAR 1993 | ALLARD M.S. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)